(19) Europäisches Patentamt
European Patent Office
Office européen des brevets

(11) Veröffentlichungsnummer: **0 341 704**
**A2**

(12) # EUROPÄISCHE PATENTANMELDUNG

(21) Anmeldenummer: **89108442.8**

(22) Anmeldetag: **10.05.89**

(51) Int. Cl.4: **C07D 403/04 , A61K 31/505 , //C07H19/067**

(30) Priorität: **12.05.88 US 192978**

(43) Veröffentlichungstag der Anmeldung: **15.11.89 Patentblatt 89/46**

(84) Benannte Vertragsstaaten: **AT BE CH DE FR GB IT LI LU NL SE**

(71) Anmelder: **F. HOFFMANN-LA ROCHE AG**
**Postfach 3255**
**CH-4002 Basel(CH)**

(72) Erfinder: **Belica, Peter Stefan**
**152 Townsend Road**
**Wanaque, N.J. 07465(US)**
Erfinder: **Huang, Tai-Nang**
**1284 Rahway Avenue**
**Westfield, N.J. 07090(US)**
Erfinder: **Manchand, Percy Sarwood**
**15 Prescott Avenue**
**Montclair, N.J. 07042(US)**
Erfinder: **Partridge, John Joseph**
**620 Airport Road**
**Chapel Hill North Carolina 27514(US)**
Erfinder: **Tam, Steve**
**13 Evergreen Road**
**West Caldwell, N.J. 07006(US)**

(74) Vertreter: **Lederer, Franz, Dr. et al**
**Patentanwalt Dr. Franz Lederer**
**Lucile-Grahn-Strasse 22**
**D-8000 München 80(DE)**

(54) **Verfahren zur Herstellung von 2',3'-dideoxycytidin.**

(57) Die Erfindung betrifft ein Verfahren zur Herstellung von 2′,3′-Dideoxycytidin, dadurch gekennzeichnet, dass man

a) die 4-Aminogruppe von Cytidin mit einer geeigneten Schutzgruppe schützt,

b) das geschützte Cytidin zum entsprechenden Bromacetatderivat bromacetyliert,

c) das Bromatom und die Acetoxygruppe aus dem Bromacetat reduktiv eliminiert um das 2′,3′-Didehydroderivat zu erhalten,

d) das 2′,3′-Didehydroderivat hydriert und

e) aus dem so erhaltenen an der 4-Amino und 5′-Hydroxygruppe geschützten 2′,3′-Dideoxycytidin die Schutzgruppen entfernt, um 2′,3′-Dideoxycytidin zu erhalten;

sowie neue in diesem Verfahren vorkommende Zwischenprodukte.

EP 0 341 704 A2

# Verfahren zur Herstellung von 2′,3′-Dideoxycytidin

Die vorliegende Erfindung betrifft ein Verfahren zur Herstellung von 2′,3′-Dideoxycytidin (ddC) und dabei verwendete neue Zwischenprodukte.

Das erfindungsgemässe Verfahren ist dadurch gekennzeichnet, dass man

a) die 4-Aminogruppe von Cytidin mit einer geeigneten Schutzgruppe schützt,

b) das geschützte Cytidin zum entsprechenden Bromacetatderivat bromacetyliert,

c) das Bromatom und die Acetoxygruppe aus dem Bromacetat reduktiv eliminiert um das 2′,3′-Didehydroderivat zu erhalten,

d) das 2′,3′-Didehydroderivat hydriert und

e) aus dem so erhaltenen an der 4-Amino und 5′-Hydroxygruppe geschützten 2′,3′-Dideoxycytidin die Schutzgruppen entfernt, um 2′,3′-Dideoxycytidin zu erhalten.

Das die Verfahrensstufen a) bis e) umfassende Verfahren ist nachstehend im Schema I dargestellt:

Schema I

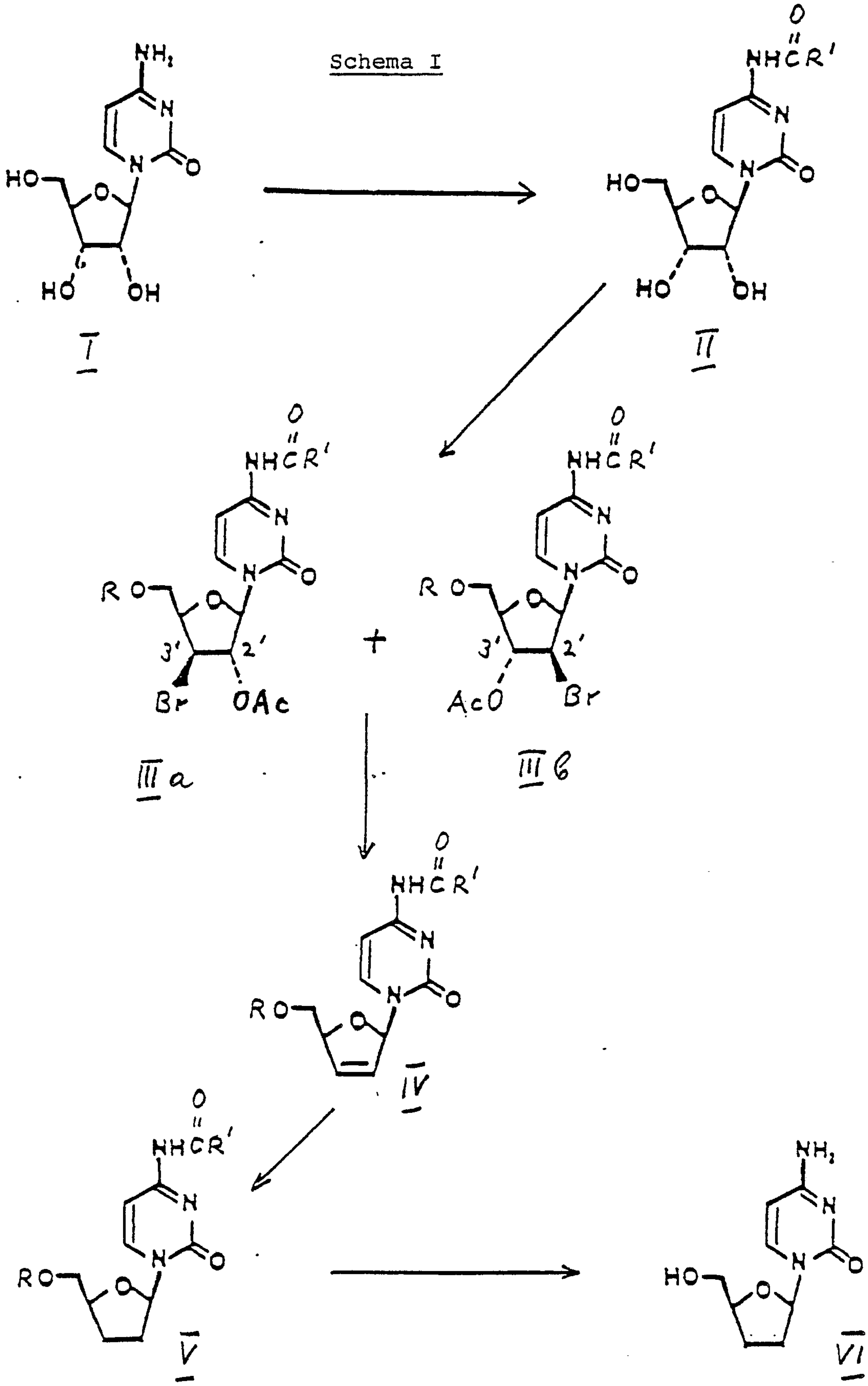

I

II

III a

III b

IV

V

VI

Im Schema I bedeutet Ac Acetyl. R′ ist substituiertes oder unsubstituiertes Niederalkyl, Aryl oder Aralkyl, wobei die Substituenten Halogen, Alkyl, Nitro oder Alkoxy sein können. R ist substituiertes oder unsubstituiertes 2-Acetoxy-2-methylpropanoyl, 2-Acetoxypropanoyl oder 2-Acetoxybenzoyl, wobei die Substituenten Niederalkyl, Aryl oder Aralkyl sein können.

Niederalkyl bezeichnet einen geradkettigen oder verzweigten Kohlenwasserstoff mit 1-7 C-Atomn. Aryl bedeutet substituiertes oder unsubstituiertes Phenyl, wobei die Substituenten Niederalkyl, Niederalkoxy, Nitro, Amino oder Halogen sein können. Niederalkoxy bedeutet eine Niederalkyläthergruppe, wobei das Alkyl wie oben definiert ist. Halogen bedeutet Chlor, Fluor, Brom oder Jod. Aralkyl bedeutet einen Alkylrest der mit einer oder mehreren Arylgruppen substituiert ist.

Die Verbindungen der Formel IIIa, IIIb, IV und V sind neu und bilden ebenfalls Gegenstand der vorliegenden Erfindung.

Im Verfahrensschritt a) wird die 4-Aminogruppe des Cytidins mit einer geeigneten Schutzgruppe in an sich bekannter Weise geschützt. Beispielsweise kann Cytidin durch Umsetzung mit Acetanhydrid in an sich bekannter Weise in N-Acetylcytidin übergeführt werden.

Verfahrensstufe b) umfasst einen neuen Bromacetylierungsschritt und liefert die Verbindung der Formel III. In einer Ausführungsform der Verfahrensstufe b) wird das geschützte Cytidin mit substituiertem oder unsubstituiertem 2-Acetoxy-2-methylpropanoylbromid, 2-Acetoxybenzoylbromid oder 2-Acetoxypropanoylbromid umsetzt. Vorzugsweise setzt man N-Acetylcytidin mit unsubstituiertem 2-Acetoxy-2-methylpropanoylbromid oder 2-Acetoxypropanoylbromid um.

In einer anderen Ausführungsform der Verfahrensstufe b) wird N-Acetylcytidin mit HBr in Essigsäure umgesetzt. Die Bromacetylierung von N-Acetylcytidin nach einer der beiden Ausführungsformen liefert ein Gemisch von Bromacetaten in hoher Ausbeute.

Verfahrensstufe c) betrifft eine reduktive Eliminierung. In einer Ausführungsform dieses Verfahrensschritts wird die Verbindung der Formel IIIa oder IIIb über Zink-Kupfer reduziert und liefert eine Verbindung der Formel IV. In einer anderen Ausführungsform der Verfahrensstufe c) kann die Verbindung der Formel IV durch elektrolytische Reduktion erhalten werden.

Verfahrensstufe d) betrifft die Hydrierung der Verbindung der Formel IV. Vorzugsweise wird die Hydrierung über Palladium/Kohle-Katalysatoren in Lösungsmittelgemischen durchgeführt die Tetrahydrofuran enthalten, vorzugsweise in Methanol und Tetrahydrofuran.

In Verfahrensschritt e) wird dann durch Entfernen der 4-Amino- und 5′Hydroxyschutzgruppen durch basische Hydrolyse ddC erhalten.

Der hier verwendete Ausdruck Zink-Kupfer bedeutet eine Kombination von Zink und Kupfer, wie sie im Beispiel VA beschrieben ist. Der Ausdruck Regioisomer bezeichnet Stellungsisomere bei denen 2 Stellungen der Verbindung vertauschbar sind. Beispielsweise sind Verbindungen der Formel IIIa und IIIb Regioisomere.

Die Erfindung wird durch die nachstehenden Beispiele weiter erläutert.

## Beispiel 1

5 g N-Acetylcytidin und 50 ml 30%-iges HBr in Essigsäure und 5 ml Acetanhydrid wurden in einem Oelbad 18 Stunden auf 50°C erwärmt. Das Reaktionsgemisch wurde auf Raumtemperatur gekühlt, in 250 ml Methylenchlorid gelöst und zweimal mit 250 ml 0,05M Kaliumphosphatpuffer, pH 7, und zweimal mit 250 ml gesättigter wässriger Natriumbicarbonatlösung gewaschen. Die wässrigen Phasen wurden mit 250 ml Methylenchlorid gewaschen. Die vereinigten organischen Phasen wurden über Natriumsulfat getrocknet und zur Trockene eingedampft. Man erhielt 6,1 g (81%) eines Gemisches von N-Acetyl-3′-brom-3′-deoxycytidin-2′,5′-diacetat und dessen 2′,3′-Regioisomer.

## Beispiel 2

Eine Suspension von 142,6 g (0,5 Mol) N-Acetylcytidin und 1,25 l Acetonitril wurden unter Stickstoff gerührt, auf 5°C gekühlt und tropfenweise mit 225 ml 2-Acetoxy-2-methylpropionylbromid im Verlauf von 30 Minuten versetzt. Dabei entstand eine homogene Lösung. Die Lösung wurde über Nacht bei Raumtemperatur gerührt (die Reaktion war innerhalb von 3 Stunden beendet), auf 5°C gekühlt und mit 1,25 l

Aethylacetat verdünnt. Danach wurden, ebenfalls bei 5° C 2 l gesättigte, wässrige Natriumbicarbonatlösung zugesetzt. Das Gemisch wurde 5 Minuten gerührt, die organische Phase wurde abgetrennt und die wässrige Phase mit 500 ml Aethylacetat rückextrahiert. Die vereinigten organischen Extrakte wurden mit 1 l gesättigter Kochsalzlösung gewaschen, über Magnesiumsulfat getrocknet und eingedampft, wobei ein Gummi erhalten wurde. Nach einstündigem Trocknen bei 40° C und 1,33 hPa wurden 264,7 g eines weissen Feststoffs erhalten. Hochdruck-Flüssigchromatographie lieferte die folgenden Resultate (nur die grösseren Peaks sind angegeben):

| Verbindung | | Relative Retentionszeit (RRT) [Min.] | % |
|---|---|---|---|
| III(a) | O<br>‖<br>$R=(CH_3)_2C-C-$, $R'=CH_3$<br>\|<br>OAc | 48.77 | 55 |
| III(b) | | 60.20 | 30 |

Chromatographiert wurde mit Methanol:Wasser (40:60) an einer $C_{18}$-Säule; der Nachweis wurde durch Messung der Absorption bei 245 nm vorgenommen.

## Beispiel 3

28,52 g N-Acetylcytidin in 250 ml Acetonitril wurde auf 10° C gekühlt und mit 48,75 g (S)(-)-2-Acetoxypropionylbromid im Verlauf von 15 Minuten versetzt. Das Gemisch wurde über Nacht bei Raumtemperatur gerührt, auf 10° C gekühlt, mit 400 ml kalter (0° C) gesättigter Natriumbicarbonatlösung versetzt und mit 250 ml Aethylacetat extrahiert. Der Extrakt wurde mit 200 ml gesättigter Kochsalzlösung gewaschen, über Magnesiumsulfat getrocknet und eingedampft, wobei 45,45 g eines weissen Schaums erhalten wurden.

Hochdruck-Flüssigchromatographie lieferte die folgenden Resultate (nur die grösseren Peaks sind angegeben):

| Verbindung | | Relative Retentionszeit (RRT) [Min.] | % |
|---|---|---|---|
| III(a) | O<br>‖<br>$R=CH_3CH-C-$, $R'=CH_3$<br>\|<br>OAc | 28.53 | 40 |
| III(b) | | 37.60 | 24 |

Reversphasenchromatographie ($C_{18}$-Säule) mit 40% Methanol in Wasser lieferte ein reines Präparat von III(a), N-Acetyl-3′-brom-3′-deoxycytidin-2′-acetat-5′-[2-(acetoxy)-propionat].

## Beispiel 4

4,5 g N-Acetylcytidin in 45 ml Methylenchlorid wurden auf 5° C gekühlt und mit 11,5 g 2-Acetoxybenzoylbromid versetzt. Danach wurde das Gemisch bei Raumtemperatur 4 Stunden gerührt, und mit 70 ml Natriumbicarbonatlösung verdünnt. Die organische Phase wurde abgetrennt und die wässrige Phase mit 50 ml Methylenchlorid extrahiert. Die vereinigten organischen Phasen wurden mit gesättigter Kochsalzlösung gewaschen, über Magnesiumsulfat getrocknet und eingedampft, wobei 10,4 g eines Schaums erhalten wurden. Chromatographie von 3,5 g dieses Produkts durch Reversphasenchromatographie ($C_{18}$-Säule) mit 40% Methanol in Wasser lieferte 12,2 g eines Gemischs von N-Acetyl-3′-brom-3′-deoxycytidin-2′-acetat-5′-[2-(acetyloxy)benzoat]-bromacetat (IIIa) und dessen Regioisomer IIIb, UV (EtOH):298 (E = 9,600), 243 (E = 20,000) and 217 (E = 17, 500) nm.

| Verbindung | Relative Retentionszeit | |
|---|---|---|
| | (RRT) [Min. ] | % |
| III(a) R′ = 2-Acetoxybenzoyl | 53.60 | 29.6 |
| III(b) R′ = $CH_3$ | 58.40 | 48.3 |

## Beispiel 5

A) 4,50 kg Zinkstaub wurden mit 3,75 l 3%-iger Salzsäure 3-5 Minuten gerührt. Die Salzsäure wurde abdekantiert, und dieser Vorgang mit 3 x 3,75 l, d.h. insgesamt 14 l 3%-iger Salzsäure wiederholt. Der Zinkstaub wurde dann mit 4 x 3 l deionisiertem Wasser gewaschen, um restliche Salzsäure zu entfernen. Nachdem das gesamte Wasser abdekantiert worden war, wurde die schwammartige Zinkschicht mit einer Lösung versetzt, die durch Lösen von 240 g Kupfer-II-Sulfat-Dihydrat in 7,5 l deionisiertem Wasser hergestellt worden war. Die Suspension wurde während des Zusatzes der Lösung rasch gerührt. Die Aquamarinfarbe der Kupfer-II-Sulfatlösung verschwand beinahe sofort und die Zinksuspension veränderte sich farblich von grau zu schwarz. Die nahezu wässrige Schicht wurde abdekantiert und der Feststoff mit 4 x 3 l deionisiertem Wasser gewaschen. Die Suspension von Zink-Kupfer wurde filtriert und nacheinander mit 4 x 3,0 l Aethanol und 3 x 3 l Aether gewaschen. Der schwarze Feststoff wurde luftgetrocknet, danach über Nacht bei 25° C und 187 hPa, danach 3 Stunden bei 130-140° C und 0,67 hPa, zur Entfernung des Aethers. Der Feststoff wurde auf Raumtemperatur unter Vakuum abgekühlt und unter Argon in braunen Flaschen aufbewahrt. Das Verfahren lieferte 3,84 kg Zink-Kupfer.

B) 1,26 kg N-Acetyl-3′-brom-3′-deoxycytidin-2′,5′-diacetat und dessen 2′,3′-Regioisomer, die wechselnde Mengen Aethanol enthielten, wurden in 4 l Acetonitril gelöst. Die Lösung wurde bei 50° C und 94 hPa zur Trockene eingedampft, um restliches Aethanol zu entfernen. 1,26 kg trockenes Bromacetat wurden in 16 l Acetonitril gelöst. Die Lösung wurde mit 350 g Zink-Kupfer versetzt, das heterogene Gemisch bei 94 hPa entgast und mit Stickstoff begast. Das Gemisch wurde zweimal entgast und mit Stickstoff begast. Ein kleines Volumen Stickstoff wurde durch die Mischung geleitet. Hochdruck-flüssigchromatographische Analyse zeigte, dass die Reaktion nach Rühren über Nacht bei Raumtemperatur beendet war. Das Reaktionsgemisch wurde durch eine Celite-Schicht abfiltriert und das Filterbett mit Acetonitril gewaschen. Danach wurden 250 ml Acetanhydrid und 250 ml Pyridin zugesetzt um deacetyliertes Produkt zu reacetylieren. Das Reaktionsgemisch wurde dann bei 50° C und 94 hPa unverzüglich konzentriert, der Rückstand aus 2 Ansätzen in 40 l Methylenchlorid gelöst und mit einer warmen Lösung von 1,5 kg Natriumbicarbonat und einer Lösung von 1,5 kg Dinatriumäthylendiamintetraacetat in 15 l deionisiertem Wasser und Erwärmen im Dampfbad auf 50° C gewaschen. Nach dem die Schichten getrennt waren, wurde eine neue Lösung von 15 l gepufferter EDTA-Lösung zu der wässrigen Phase gegeben, die wässrige Phase mit 40 l Methylenchlorid unter Rühren während 15 Minuten extrahiert, die organische Phase abgetrennt und die wässrige Phase von Unlöslichem abfiltriert. Die Feststoffe wurden in 15 l warmer gepufferter EDTA-Lösung suspendiert und mit 40 l Methylenchlorid unter raschem Rühren während 15 Minuten extrahiert. Alle organischen Phasen wurden nacheinander mit 2 x 15 l warmer gepufferter EDTA-Lösung und 20 l gesättigter Natriumbicarbonatlösung gewaschen. Die Phasen wurden getrennt und die organischen Phasen in sechs 20 l Flaschen gesammelt. Ein Teil der Flaschen wurden über Natriumsulfat, das etwas Kohle enthielt unter Rühren getrocknet. Das Gemisch wurde durch ein Celite-Filterbett filtriert und bei 50° C und 94 hPa konzentriert, wobei ein dunkler halbfester Stoff erhalten wurde. Dieser Stoff wurde mit 4 l kaltem Tetrahydrofuran und 2 l

Petroläther behandelt, der gelbliche Feststoff zur Gewichtskonstanz getrocknet, wobei 986 g N-Acetyl-2′,3′-didehydro-2′,3′-dideoxycytidin-5′-acetat erhalten wurden. Hochdruck-Flüssigchromatographie zeigte, dass das Material 93% rein und geeignet für den nächsten Verfahrensschritt war.

Die Ausbeuten der anderen Reaktionsansätzen variierten von 45-55%. Ein Analysenpräparat wurde durch Umkristallisation aus Tetrahydrofuran unter Erhalt eines weissen Feststoffes vom Schmelzpunkt >350° erhalten; $[\alpha]_D^{25}$ = +15,8° (c = 0,33, DMS0); Literatur Schmelzpunkt >280° [T. Adachi, T. Iwasaki, I. Inoue, and M. Miyoshi, J. Org. Chem., 44, 1404 (1979)].

## Beispiel 6

259,0 g eines Gemisches der Bromacetate aus Beispiel 2 in 2,5 l Acetonitril wurden durch mehrfaches Evakuieren und anschliessendes Auffüllen des Reaktionsgefässes mit Argon vom Sauerstoff befreit. Danach wurden 100 g Zink-Kupfer (aus Beispiel 5A) zugesetzt und das Gemisch wurde bei Raumtempe-ratur über Nacht unter Argon gerührt. Danach wurde über Celite abfiltriert, das Reaktionsgefäss mit 200 ml Acetonitril gespühlt und mit dieser Lösung das Filterhilfsmittel gewaschen. Die vereinigten Filtrate und Waschwässer wurden bei 40° C eingedampft und der Rückstand in 1,25 l Methylenchlorid gelöst. Die Lösung wurde zu einer Lösung von 200 g Aethylendiamintetraessigsäure-Dinatriumsalzdihydrat in 2 l deionisiertem Wasser, das 200 g Natriumbicarbonat enthielt, gegeben. Das Gemisch wurde 1,5 Stunden kräftig gerührt, über Celite filtriert und das Filterhilfsmittel mit 300 ml Methylenchlorid gewaschen. Die organische Phase wurde abgetrennt und die wässrige Phase mit 500 ml Methylenchlorid rückextrahiert. Die vereinigten organischen Phasen wurden mit 250 ml gesättigter Natriumbicarbonatlösung gewaschen, danach mit 100 ml Methylenchlorid rückextrahiert. Danach wurden die organischen Phasen über Magnesiumsulfat getrocknet, filtriert und auf etwa 800 ml eingeengt. Dazu wurden 30 ml Acetanhydrid und anschliessend 40 g Poly-4-vinylpyridin gegeben und das Gemisch unter Stickstoff 3 Stunden gerührt. Danach wurde über Celite filtriert, das Gemisch mit 200 ml Methylenchlorid gewaschen, Filtrat und Waschwässer eingedampft, 250 ml Toluol zugesetzt und das Gemisch erneut eingedampft. Danach wurden 500 ml Aether zugegeben und es wurde 15 Minuten kräftig gerührt. Das Gemisch wurde abfiltriert, mit 200 ml Aether gewaschen. Man erhielt 143,3 g eines Gemisches von N-Acetyl-2′,3′-didehydro-2′,3′-dideoxycytidin-5′-acetat und N-Acetyl-2′,3′-didehydro-2′,3′-dideoxycytidin-5′-[(2-acetoxy-2-methyl)propionat] als gelblichen Feststoff.

Hochdruck-Flüsigchromatographie

| Verbindung | Relative Retentionszeit (RRT) [Min.] | % |
| --- | --- | --- |
| IV, R=$CH_3\overset{O}{\overset{\|}{C}}$-, R'=$CH_3$ | 5.83 | 6 |
| VI, R=$(CH_3)_2$-$\underset{OAc}{\underset{\|}{C}}$-$\overset{O}{\overset{\|}{C}}$-, R'=$CH_3$ | 13.50 | 91 |

Umkristallisation des Reaktionsgemisches aus heissem Tetrahydrofuran lieferte reines N-Acetyl-2′,3′-didehydro-2′,3′-dideoxycytidin -5′-[(2-acetoxy-2-methyl)propionat], Schmelzpunkt 173-175° C; $[\alpha]_D^{25}$ + 123,5° C (c = 1,0; $CHCl_3$).

## Beispiel 7

1,47 g eines Gemisches der Bromacetate aus Beispiel 3 wurde wie in Beispiel 7 beschrieben mit 800 mg Zink-Kupfer reduziert und lieferte 570 mg N-Acetyl-2′,3′-didehydro-2′,3′-dideoxycytidin-5′-[(2-acetoxy)-propionat] nach Umkristallisation aus heissem Tetrahydrofuran, Schmelzpunkt 125° C; $[\alpha]_D^{25}$ = +119,04 (c = 0,25; $CHCl_3$).

## Beispiel 8

30,5 g eines Gemisches der Bromacetate von Beispiel 4 wurde durch Evakuierung und Abfüllen des Reaktionsgefässes mit Argon vom Sauerstoff befreit; dieses Verfahren wurde zweimal wiederholt. Danach wurden 10 g Zink-Kupfer (aus Beispiel 5A) zugesetzt und das Gemisch wurde unter Argon bei Raumtemperatur über Nacht gerührt. Danach wurde über Celite abfiltriert, das Reaktionsgefäss mit 20 ml Acetonitril ge spühlt und mit der Spühlflüsigkeit das Filterhilfsmittel gewaschen. Die vereinigten Filtrate und Waschwässer wurden eingedampft und der Rückstand in 125 ml Methylenchlorid gelöst. Dieser wurde dann zu einer Lösung von 20 g Aethylendiamintetraessigsäure-Dinatriumsalzdihydrat in 200 ml Wasser, die 20 g Natriumbicarbonat enthielten gegeben. Das Gemisch wurde 1,5 Stunden bei Raumtemperatur gerührt und über Celite filtriert. Die organische Phase wurde abgetrennt und die wässrige Phase mit 75 ml Methylenchlorid rückextrahiert. Die vereinigten organischen Phasen wurden über Magnesiumsulfat getrocknet und filtriert. Das Filtrat wurde mit 6,0 ml Acetanhydrid und 850 mg 4-Dimethylaminopyridin versetzt. Das Gemisch wurde 2 Stunden bei Raumtemperatur gerührt, mit 25 ml Aethanol versetzt und dann auf etwa 100 ml eingeengt. Danach wurden 25 ml Toluol zugesetzt und das Gemisch zur Trockene eingedampft. Der Rückstand wurde aus 100 ml Aether-Aethanol (1:1) umkristallisiert und lieferte 6,3 g N-Acetyl-2′,3′-didehydro-2′,3′-dideoxycytidin-5′-[2-(acetoxy)benzoat]. Umkristallisation aus heissem Aethanol:Tetrahydrofuran (1:1) lieferte ein Analysenpräparat vom Schmelzpunkt 265° C (Zers.), UV (EtOH): 299 (E = 9,800), 243 (E = 21,550), und 203 (E = 48,400) nm.

## Beispiel 9

In das Kathodenreservoir einer elektrolytischen Zelle wurden 35 g N-Acetyl-3′-brom-3′-deoxycytidin-2′,5′-diacetat und 1 l 0,25M Tetraäthylammoniumtosylat in Acetonitril gegeben. In das Anodenreservoir wurden 1 l 0,025M Tetraäthylammoniumtosylat in Acetonitril gegeben. Kathoden- und Anodenflüssigkeit wurden durch die elektrolytische Zelle mit einer Flussgeschwindigkeit von 200 ml/Minute zirkulieren gelassen. Die Zelle war unterteilt durch eine Anionenaustauschermembran und die anfängliche Stromdichte betrug 2,4 $mA/cm^2$ bei -1,5V. Der Reaktionsablauf wurde durch Dünnschichtchromatographie und Hochdruck-flüssigchromatographie verfolgt. Während der ersten 8 Stunden fiel das ge wünschte Produkt, N-Acetyl-2′,3′-dideoxycytidin-5′-acetat, aus dem Reaktionsgemisch aus. Das Produkt wurde abfiltriert und die Reaktion fortgesetzt. Nach 16 Stunden war die Reaktion fast vollständig. Die Kathodenflüssigkeit wurde gesammelt und bei Raumtemperatur und 13,3 hPa eingedampft. Der trockene Rückstand wurde mit den vorher abfiltrierten Feststoffen vereinigt. Das Gemisch von Produkt und Elektrolyt wurde dann in 200 ml deionisiertem Wasser gelöst. Das Gemisch wurde mit 3 x 300 ml Methylenchlorid extrahiert, der organische Extrakt über wasserfreiem Natriumsulfat getrocknet und zur Trockene eingedampft. Der Rückstand wurde mit 180 ml Tetrahydrofuran behandelt und lieferte 18,12 g (76% Ausbeute) N-Acetyl-2′,3′-didehydro-2′,3′-dideoxycytidin-5′-acetat (IV, R = $CH_3CO$, R′ = $CH_3$), Schmelzpunkt >300° C (Zers.), $[\alpha]_D^{25}$ + 14,7° (c = 0,387, DMSO). Das Produkt war gemäss der Hochdruckflüssigchromatographie 99,2% rein.

## Beispiel 10

In den Kathodenraum einer elektrolytischen Zelle wurden 10,0 g N-Acetyl-3′-bromo-3′-deoxycytidin-5′-[-(2-acetoxy -2-methyl)propionat] und 500 ml 0,25M Tetraäthylammoniumtosylat in Acetonitril gegeben. In den Anodenraum wurden 500 ml 0,025M Tetraäthylammoniumtosylat in Acetonitril gegeben. Die Elektrolytflüssigkeit wurde durch die Zelle mit einer Fliessgeschwindigkeit von 250 ml/Minute zirkulieren gelassen.

Die Zelle war durch eine Ionenaustauschermembran (Ionac MA-3475, Sybron Chemical Division, Birmingham, NJ) unterteilt und die Anfangsstromdichte betrug 0,8 mA/cm² bei -4,0V. Die Reaktion wurde durch Dünnschichtchromatographie und Hochdruck-flüssigchromatographie verfolgt. Während der ersten 30 Minuten fand ein 60%-iger Umsatz statt. Etwa 90 Minuten später zeigte die Dünnschichtchromatographie, dass das gesamte Ausgangsmaterial verschwunden war. Die Kathodenflüssigkeit wurde gesammelt und bei Raumtemperatur unter vermindertem Druck zur Trockene eingedampft. Der trockene Rückstand wurde dann in 200 ml entionisiertem Wasser gelöst. Die Lösung wurde mit 3 x 200 ml Methylenchlorid extrahiert, der organische Extrakt über Natriumsulfat getrocknet und zur Trockene eingedampft. Man erhielt 3,07 g gelblichen Feststoff (42% Ausbeute). 0,5 g dieses Produktgemischs wurden in 8 ml heissem Tetrahydrofuran gelöst und über Nacht zur Kristallisation stehen gelassen. Die feste Kristallmasse wurde abfiltriert und mit 10 ml Aether gewaschen, wobei 0,22 g N-Acetyl-2′,3′-didehydro-2′,3′-dideoxycytidin-5′-[(2-acetoxy -2-methyl)propionat] erhalten wurden. Umkristallisation aus heissem Tetrahydrofuran lieferte Kristalle vom Schmelzpunkt 170-172° C.

## Beispiel 11

Zu 16 l Tetrahydrofuran und 22 l Methanol wurde eine Aufschlämmung von 500 g N-Acetyl-2′,3′-didehydro-2′,3′-dideoxycytidin-5′-acetat in 1,5 l Methanol gegeben. Das Vorratsgefäss wurde mit 500 ml Methanol nachgewaschen und die Lösung in das Reaktionsgefäss gegeben. Das Reaktionsgemisch wurde dann mit Argon überschichtet, mit 20 g 10% Palladium/Kohle-Katalysator in 200 ml Methanol versetzt. Das Gefäss wurde dann auf 94 hPa evakuiert und mit Wasserstoff gefüllt. Der Evakuierungs-Füllprozess wurde dreimal vorgenommen. Danach wurde ein Wasserstoffdruck von einer Atmosphäre aufrecht erhalten und das Gemisch wurde bei Raumtemperatur gerührt. Nach 52 Minuten klang die Reaktion deutlich ab, das Reaktionsgefäss wurde auf 94 hPa evakuiert und mit Argon gefüllt. Dieses Verfahren wurde dreimal ausgeführt. Das Reaktionsgemisch wurde dann vorsichtig über ein Filterhilfsmittel filtriert, dieses mit 500 ml ca. 40% Tetrahydrofuran in Methanol gewaschen und das Filtrat bei 50° und 94 hPa zur Trockene eingedampft. Auf diese Weise wurden 9 Ansätze hydriert wobei N-Acetyl-2′,3′-dideoxycytidin-5′-acetat mit einer kleinen Menge N-Acetylcytosin als Nebenprodukt erhalten wurden. Das Filtrat wurde bei 70-80° und 94 hPa konzentriert und lieferte einen weissen Feststoff. Der Feststoff wurde mit 4,5 l Acetonitril 15 Minuten gerührt, die Paste auf -10° während 15 Minuten abgekühlt und filtriert, wobei in Acetonitril lösliche Verunreinigungen (kleine Mengen überhydrierter Produkte, wie 5,6-Dihydro-2′,3′-dideoxyuridin-5′-acetat) entfernt wurden. Der weisse Filterkuchen wurde dann in 20 l heissem Acetonitril unter Bildung einer trüben Lösung gelöst, die durch Celite filtriert wurde, um unlösliches N-Acetylcytosin zu entfernen. Das farblose Filtrat wurde bei 70-80° und 94 hPa auf 10 l konzentriert. Die Lösung wurde auf 10° eine Stunde lang gekühlt um die Kristallisation herbeizuführen. Der Feststoff wurde auf einem Büchnertrichter gesammelt und mit 4 l kaltem Acetonitril gewaschen. Der Feststoff wurde dann bei 80° getrocknet und lieferte 3,3 kg (66%) einer ersten Fraktion von N-Acetyl-2′,3′-dideoxycytidin-5′-acetat, Schmelzpunkt 210-211°, $[\alpha]_D^{25} = +92,0°$ (c = 0,49, $CH_3OH$).

Die Mutterlaugen und Waschwässer wurden aus mehreren Ansätzen gesammelt und konzentriert. Umkristallisation dieses Materials lieferte zusätzliche 10-16% N-Acetyl-2′,3′-dideoxycytidin-5′-acetat vergleichbarer Qualität. Die Mutterlaugen aus dieser zweiten Fraktion enthielten kleine Mengen 5,6-Dihydro-2′,3′-dideoxyuridin-5′-acetat. Reinigung dieser Mutterlaugen an Silicagel mit 10:1 Aethylacetat-Methanol lieferte weitere 3-5% des gewünschten Produkts.

## Beispiel 12

142,3 g der Bromacetate von Beispiel 6 in 800 ml Methanol wurden bis zur Lösung erwärmt, mit 800 ml Tetrahydrofuran verdünnt und dann auf Raumtemperatur gekühlt. Danach wurden 8,9 g 10% Palladium/Kohle unter Argon zugesetzt und das Gemisch bei Raumtemperatur und Atmosphärendruck bis zum Ende der Wasserstoffaufnahme hydriert (11 l ca. 3 Stunden). Das Gemisch wurde über Celite filtriert und das Filterhilfsmittel mit 300 ml Methanol gewaschen. Die vereinigten Filtrate und Waschwässer wurden eingedampft und lieferten 132,7 g eines Gemisches von N-Acetyl-2′,3′-di deoxycytidin-5′-acetat und N-Acetyl-2′,3′-dideoxycytidin-5′-[(2-acetoxy -2-methyl)propionat].

Hochdruckflüssigchromatographie:

<table>
<tr><th>Verbindung</th><th>Relative Retentionszeit (RT) [Min.]</th><th>%</th></tr>
<tr><td>V $R=CH_3$, $R'=CH_3$</td><td>7,40</td><td>6</td></tr>
<tr><td>V O<br>||<br>$R=(CH_3)_2$-C-C-, $R'=CH_3$<br>|<br>OAc</td><td>17,93</td><td>90</td></tr>
<tr><td>N-Acetylcytosin</td><td>3,67</td><td>1</td></tr>
</table>

Ein reines Präparat von N-Acetyl-2′,3′-dideoxycytidin-5′-[(2-acetyl -2-methyl)propionat] wurde als Schaum durch Chromatographie an Silicagel mit 1% Methanol in Methylenchlorid erhalten. $[\alpha]_D^{25}$ + 136,08 ($CHCl_3$, c = 1,02).

## Beispiel 13

Eine Lösung von 720 mg des Produkts aus Beispiel 7 in 10 ml Methanol und 10 ml Tetrahydrofuran wurde über 200 mg 10% Palladium/Kohle bei Raumtemperatur und Atmosphärendruck bis zur Beendigung der Wasserstoffaufnahme (10 ml) hydriert. Das Gemisch wurde über Celite filtriert und das Filtrat zu einem Gummi eingedampft. Chromatographie an 10 g Silicagel mit 10% Methanol in Methylenchlorid lieferte 290 mg N-Acetyl-2′,3′-dideoxycytidin-5′-[(2-acetoxy -2-methyl)-propionat als Schaum, $[\alpha]_D^{25}$ + 88,43 (c = 0,99, $CHCl_3$); UV (EtOH): 299 (E = 6,420), 246 (E = 1241), und 214 (E = 16,500) nm.

## Beispiel 14

Eine Lösung von 20,7 g des Produkts aus Beispiel 13 in 100 ml Aethanol wurde mit 10,0 ml Triton B (N-Benzyltrimethylammoniumhydroxid) behandelt. Das Gemisch wurde über Nacht bei Raumtemperatur gerührt, auf 20 ml konzentriert, auf 0° gekühlt und filtriert. Der Rückstand wurde mit 10 ml kaltem Aethanol gewaschen und lieferte 4,48 g 2′,3′-Dideoxycytidin vom Schmelzpunkt 215-218° C als weissen Feststoff.

## Beispiel 15

Zu einem 1,66 g des Produkts von Beispiel 8 in 30 ml Methanol und 30 ml Dimethylformamid wurden 300 mg 10% Palladium/Kohle unter Argon gegeben. Das Gemisch wurde bis zur Beendigung der Wasserstoffaufnahme (113 ml) bei Raumtemperatur und Atmosphärendruck hydriert. Das Gemisch wurde über Celite abfiltriert und das Filtrat unter vermindertem Druck zu einem Gummi eingedampft, der an 15 g Silicagel mit 2% Methanol in Methylenchlorid als Elutionsmittel chromatographiert wurde. Man erhielt 970 mg N-Acetyl-2′,3′,-dideoxycytidin-5′-[2-(acetoxy)benzoat] als weissen Schaum; UV (EtOH); 298 (E = 9,450) 243 (E = 19,500), und 203 (E = 42,350) nm.

## Beispiel 16

Eine Lösung von 33,4 g des Produkts von Beispiel 15 in 330 ml Methanol, 33 ml Wasser und 66 ml Triäthylamin wurde unter Argon 7 Stunden bei 65° C und dann über Nacht bei Raumtemperatur gerührt. Das Reaktionsgemisch wurde unter vermindertem Druck eingedampft und der Rückstand dreimal mit Toluol abgedampft. Der Rückstand wurde in 50 ml Aethanol gelöst, mit 400 ml Aceton zersetzt und über Nacht bei Raumtemperatur gerührt. Das Produkt wurde abfiltriert wobei 4,04 g 2′,3′-Dideoxycytidin, Schmelzpunkt 218-220° C erhalten wurden.

Beispiel 17

1,55 kg N-Acetyl-2′,3′-dideoxycytidin-5′-acetat, 10,6 ml Methanol, 3,1 l Triäthylamin und 1,56 l entionisiertes Wasser wurden auf 60-65° (Badtemperatur) erwärmt. Das Gemisch wurde 3 Stunden bei 55-60° gerührt, dann 12 Stunden bei Raumtemperatur. Das Reaktionsgemisch wurde unter vermindertem Druck bei 70-80° auf ein Volumen von 2,5 l konzentriert. Danach wurden 1,5 l absolutes Aethanol zugesetzt und das Gemisch wiederum unter vermindertem Druck auf 2,5 l konzentriert um restliches Lösungsmittel zu entfernen. Die halbfeste Masse wurde eine halbe Stunde auf 10° gekühlt, dann abfiltriert und mit 1,5 l absolutem Aethanol gewaschen. Der Feststoff wurde aus 50 l absolutem Aethanol umkristallisiert. Das Filtrat wurde unter vermindertem Druck bei 70-80° auf ein Volumen von 3 l konzentriert. Der Feststoff wurde 10 Minuten zum Rückfluss erhitzt, dann auf 10° während 2 Stunden gekühlt und das Kristallisat abfiltriert, und mit 2 l absolutem Aethanol gewaschen. Der Rückstand wurde bei 85° und 1,33 hPa über Nacht getrocknet und lieferte 1,02 kg (92%) 2′,3′-Dideoxycytidin als weissen Feststoff vom Schmelzpunkt 225-228° C; $[\alpha]_D^{25}$ + 76,9° (c 0,56, Wasser); $[\alpha]_D^{25}$ + 105, 0° (c 0, 50, $CH_3OH$); Lit. Schmelzpunkt 215-217°; $[\alpha]_D^{25}$ + 81° (c 0,635, Wasser) [J.P. Horowitz, J. Chua, M. Noel, und J. T. Donatti, J. Org. Chem., 32, 817 (1967)].

Die Mutterlaugen aus 3 Ansätzen wurden gesammelt und auf ein Volumen von 1 l konzentriert. Der auskristallisierte Feststoff wurde aus 5 l absolutem Aethanol umkristallisiert und lieferte weitere 2-4% 2′,3′-Dideoxycytidin.

Beispiel 18

27,2 g des Gemischs der Verbindungen von Beispiel 12 in 71 ml Methanol wurden bei Raumtemperatur bis zur Lösung gerührt und dann mit 7,14 ml Triton B (40% Benzyltrimethyl-ammoniumhydroxid in Methanol) versetzt. Danach wurde über Nacht bei Raumtemperatur weitergerührt und das Produkt filtriert. Es wurde mit etwas kaltem Methanol gewaschen und lieferte 8,33 g rohes 2′,3′-Dideoxycytidin mit einer Reinheit von 99,17% (HPLC). Eindampfen des Filtrats und der Waschwässer gab ein halbfesten Stoff der mit 20 ml Aethanol versetzt wurde. Danach wurde abfiltriert, mit etwas kaltem Aethanol gewaschen und man erhielt weitere 1,05 g rohes (96,67% Reinheit) 2′,3′-Dideoxycytidin, insgesamt also 9,38 g rohes 2′,3′-Dideoxycytidin (43,1% bezogen auf N-Acetylcytidin).

Diese 9,38 g Rohprodukt wurden in ein Gemisch von 100 ml heissem absolutem Aethanol und 12 ml entionisiertem Wasser unter Rückflusserhitzen gelöst. Die heisse Lösung wurde filtriert, der Trichter mit 10 ml Aethanol gewaschen. Die vereinigten Filtrate und Waschwässer wurden auf Raumtemperatur abkühlen gelassen und weiter auf etwa 7° C (Eisbad) gekühlt. Das Produkt wurde abfiltriert und mit wenig Methanol gewaschen, wobei 7,17 g 2′,3′-Dideoxycytidin als weisse Kristalle vom Schmelzpunkt 219-221° C, $[\alpha]_D^{25}$ + 95,86° (c = 1,46, $CH_3OH$) erhalten wurden.

**Ansprüche**

1. Verfahren zur Herstellung von 2′,3′-Dideoxycytidin, dadurch gekennzeichnet, dass man
   a) die 4-Aminogruppe von Cytidin mit einer geeigneten Schutzgruppe schützt,
   b) das geschützte Cytidin zum entsprechenden Bromacetatderivat bromacetyliert,
   c) das Bromatom und die Acetoxygruppe aus dem Bromacetat reduktiv eliminiert um das 2′,3′-Didehydroderivat zu erhalten,
   d) das 2′,3′-Didehydroderivat hydriert und

e) aus dem so erhaltenen an der 4-Amino und 5'-Hydroxygruppe geschützten 2',3'-Dideoxycytidin die Schutzgruppen entfernt, um 2',3'-Dideoxycytidin zu erhalten.

2. Verfahren nach Anspruch 1, worin man die Bromacetylierung mit substituiertem oder unsubstituiertem 2-Acetoxy-2-methylpropanoylbromid, 2-Acetoxybenzoylbromid oder 2-Acetoxypropanoylbromid, wobei die Substituenten Niederalkyl, Aryl oder Aralkyl sein können, oder mit Bromwasserstoff in Essigsäure durchführt.

3. Verfahren nach Anspruch 1, worin man die Bromacetylierung mit 30%-igem Bromwasserstoff in Essigsäure durchführt.

4. Verfahren nach Anspruch 1, worin man die Hydrierung über einen Palladium/Kohle-Katalysator in Methanol und Tetrahydrofuran durchführt.

5. Verfahren nach Anspruch 1, worin man das Bromatom und die Acetoxygruppe durch elektrolytische Reduktion aus dem Bromacetat eliminiert, um das entsprechende 2',3'-Didehydroderivat zu erhalten.

6. Verbindung der Formel

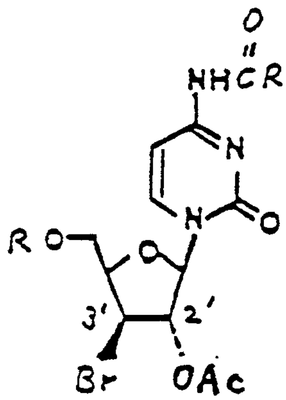

und deren Regioisomere

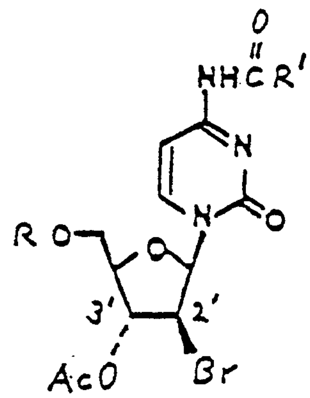

III(a)

III(b)

und die Stereoisomere davon,
worin R' substituiertes oder unsubstituiertes Niederalkyl, Aryl oder Aralkyl ist, wobei die Substituenten Halogen, Alkyl, Nitro oder Alkoxy sein können, und R substituiertes oder unsubstituiertes 2-Acetoxy-2-methylpropanoyl, 2-Acetoxypropanoyl oder 2-Acetoxybenzoyl ist, wobei die Substituenten Niederalkyl, Aryl oder Aralkyl sein können.

7. Verbindung der Formel

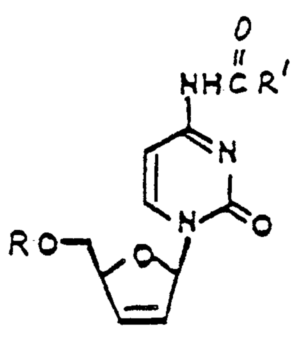

IV

worin R und R' die gleiche Bedeutung wie in Anspruch 6 haben.

8. Verbindung der Formel

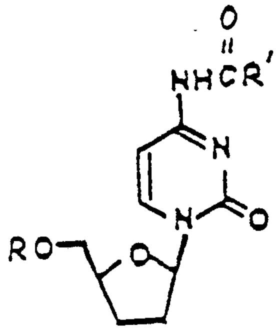

V

worin R und R' die gleiche Bedeutung wie in Anspruch 6 haben.

9. 2',3'-Dideoxycytidin wenn hergestellt nach dem Verfahren gemäss einem der Ansprüche 1-5.